# EUROPEAN PATENT SPECIFICATION

(11) **EP 2 679 573 B1**
(45) Date of publication and mention of the grant of the patent: **27.12.2017**
(21) Application number: 12382267.8
(22) Date of filing: 29.06.2012
(51) Int. Cl.: C07C 68/08, C07C 68/06, C07C 69/96, B01D 3/14, C08G 64/30

(54) **Method and apparatus for the separation of dialkyl carbonate, water and alkanol**
Verfahren und Vorrichtung zur Trennung von Dialkylcarbonat, Wasser und Alkanol
Procédé et appareil pour la séparation de diakyle carbonate, eau et alcane

(43) Date of publication of application: 01.01.2014
(73) Proprietor: SABIC Global Technologies B.V., 4612 PX Bergen op Zoom (NL)
(72) Inventor: Vic Fernandez, Ignacio, 30151 Murcia (ES); Ferrer Nadal, Sergio, 30390 Murcia (ES); Caballero, Jose Antonio, 03540 Alicante (ES); Javaloyes Antón, Juan, 03201 Alicante (ES)
(74) Representative: Carpintero Lopez, Francisco

(56) References cited:
- US-A- 5 527 943
- US-A1- 2009 137 832
- US-A1- 2010 010 252
- US-A1- 2010 261 928
- US-A1- 2012 101 247
- ÖMER YILDIRIM ET AL: "Dividing wall columns in chemical process industry: A review on current activities", SEPARATION AND PURIFICATION TECHNOLOGY, vol. 80, no. 3, 1 August 2011 (2011-08-01) , pages 403-417, XP055043075, ISSN: 1383-5866, DOI: 10.1016/j.seppur.2011.05.009

## Description

### BACKGROUND

The present disclosure relates generally to a method and an apparatus for the separation of dialkyl carbonate (" DAC"), water, and alkanol, and especially to a method and an apparatus for the production and separation of dimethyl carbonate ("DMC").

Polycarbonates are useful materials valued for their physical and optical properties. Methods for the preparation of polycarbonates include interfacial processes and melt processes. In melt processes, bisphenol can react with diaryl carbonate. Melt processes are desirable because they can avoid the use of phosgene and solvents.

Use of melt processes for polycarbonate synthesis requires an industrially efficient process for producing diaryl carbonate, such as diphenyl carbonate ("DPC"). During the production of diaryl carbonate, dialkyl carbonate can be reacted with, for example, aryl hydroxide as disclosed in U.S. Patent No. 4,182,726. Thus, efficient processes for the production of dialkyl carbonate also are needed in order to effectively produce polycarbonates. The production of dialkyl carbonate, such as DMC, typically involves a series of distillation steps using multiple distillation columns. For example, U.S. Patent Nos. 5,527,943, 7,514,521, and 7,803,961 disclose processes for preparing dialkyl carbonate. Despite these processes, however, there is a continued need for methods and apparatuses exhibiting reduced energy consumption and requiring less processing equipment, specifically fewer distillation columns, while also capable of producing purified dialkyl carbonate, especially purified dimethyl carbonate.

U.S. Patent 5,527,943 discloses a method for enabling HCl and possible entrained CuCl particulates to be removed from a gas-vapor stream leaving a dimethylcarbonate synthesis reactor.

U.S. Patent Application 2010/0010252 discloses a process for preparing diaryl carbonates from dialkyl carbonates and aromatic hydroxyl compounds using at least two reaction columns, a process section for recovering the dialkyl carbonate used in the reaction and for removing the alcohol of reaction, one or more process steps for removing the by-products obtained in the process which have a boiling point between that of the dialkyl carbonate and that of the alkyl aryl carbonate formed during the preparation of the diaryl

carbonate, and a process step for further purification of the diaryl carbonate obtained from the reaction columns.

U.S. Patent Application 2009/0137832 discloses a process comprising transesterifying a dialkyl carbonate and an aromatic hydroxyl compound in the presence of a transesterification catalyst to provide a diaryl carbonate product comprising the transesterification catalyst as an impurity; subjecting the diaryl carbonate product to distillation in a first distillation column having an upper part and a lower part, wherein the upper part comprises a rectifying section and the lower part comprises a stripping section; and withdrawing a first sidestream from the first distillation column, wherein the first sidestream comprises a purified diaryl carbonate.

U.S. Patent Application 2012/0101247 a process for continuously preparing diaryl carbonates from dialkyl carbonates and at least one monohydroxyl compound in the presence of catalysts, and to the use thereof for preparation of polycarbonates.

U.S. Patent Application 2010/0261928 discloses a process for preparing diaryl carbonates and/or alkyl aryl carbonates from dialkyl carbonates and aromatic hydroxy compounds using a reactive dividing wall column.

### BRIEF DESCRIPTION

Disclosed, in various embodiments, are methods and apparatuses for the separation of dialkyl carbonate, water, and alkanol.

In an inventive embodiment, a method of producing a purified dialkyl carbonate is provided, comprising: introducing a stream (412) comprising dialkyl carbonate, water and alkanol to a first divided wall distillation column (200); and separating the mixture with use of the divided wall distillation column (200); and recovering a product stream (426) comprising the purified dialkyl carbonate from a bottom end of the divided wall distillation column (200); recovering a first stream (431) comprising dialkyl carbonate, water, and alkanol from a top end of the divided wall distillation column (200); recovering a second stream (416) comprising dialkyl carbonate, water, and alkanol from a side of the divided wall distillation column (200);introducing the second stream (416) into a separator (S610); recovering from a bottom of the separator (S610) a third stream (418) comprising dialkyl carbonate; and recycling the third stream (418) to the side of the divided wall distillation column (200).

In an embodiment, a method of producing dialkyl carbonate can comprise: reacting oxygen, alkanol, carbon monoxide, hydrochloric acid and a catalyst in a reactor to produce an alkanol stream; separating constituents of the alkanol stream in a separator; and recovering a dialkyl carbonate stream. The method also comprises introducing the dialkyl carbonate stream to an acid removal distillation column; and recovering from the acid removal distillation column a reactant stream comprising a mixture of dialkyl carbonate, water and alkanol. The method further comprises introducing the reactant stream to a first divided wall distillation column; separating the mixture with use of the divided wall distillation column; and recovering a product stream comprising dialkyl carbonate.

In an inventive embodiment, an apparatus configured for separating a mixture comprising dialkyl carbonate, water and alkanol is provided, comprising: the first dividing wall distillation column (200) is connected to the input line for introduction of the feed, and to a first line, a second line and the product line, the first line extends from the top of the first dividing wall distillation column (200) to provide a first stream (431) comprising dialkyl carbonate, water, and alkanol, the second line extends from the side of the first dividing wall distillation column (200) to the separator (S610) to provide a second stream (416) comprising dialkyl carbonate, water and alkanol, and the product line extends from the bottom of the first dividing wall distillation column (200) to provide a product stream (426) comprising a purified dialkyl carbonate;a separator (S610) connected to the divided wall distillation column (200); and a water recovery distillation column (630) connected to the separator (S610); wherein the apparatus comprises a plurality of lines configured to transport streams between at least two of the first divided wall distillation column (200), the separator (S610) and the water recovery distillation column (630);wherein the separator (S610) is connected to a third line extends from the bottom of the separator (S610) to the first dividing wall distillation column (200) to provide a third stream (418) comprising dialkyl carbonate.

The above described and other features are exemplified by the following figures and detailed description.

### BRIEF DESCRIPTION OF THE DRAWINGS

Refer now to the Figures, which are exemplary and non-limiting embodiments, and wherein the like elements are numbered alike.
FIG. 1 schematically depicts a dialkyl carbonate production/recovery plant design.
FIG. 2 schematically depicts a plant design for the separation of DMC, water and methanol.
FIG. 3 depicts a ternary composition diagram of a DMC/methanol/water ternary system.

### DETAILED DESCRIPTION

The disclosure herein is directed to how to improve the overall efficiency, reduce energy consumption, and reduce the overall cost associated with the separation of DMC and resultant production of purified DMC (e.g., DMC having a purity of greater than 99.9 wt%). In exemplary embodiments, the number of sequential distillation columns needed to separate and purify DMC by using a divided wall distillation column can be reduced as described in further detail below. In other exemplary embodiments, in order to effectively employ a divided wall distillation column for such separation and purification, a proper concentration range of the reactants in the effluent stream entering the divided wall distillation column is identified and achieved in order to result in a desired separation of the constituents with use of the divided wall distillation column.

A more complete understanding of the components, processes, and apparatuses disclosed herein can be obtained by reference to the accompanying drawings. These Figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the exemplary embodiments. Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

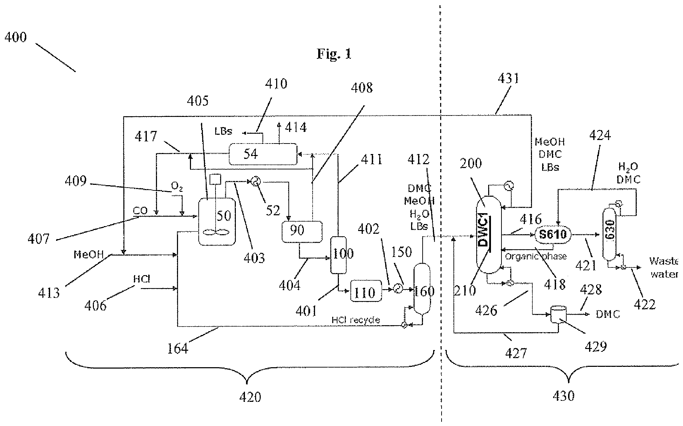

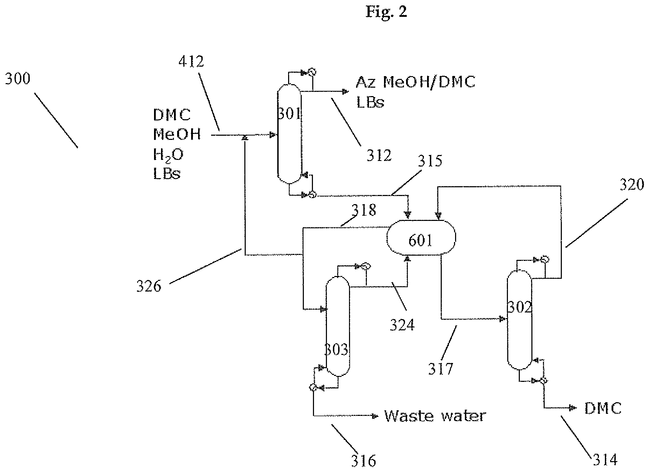

Various feed, product and recycle streams indicated numerically are shown in FIGs. 1 and 2. It is further noted that it will be appreciated by persons skilled in the art that the positioning of the various streams/lines as described herein as being, e.g., in the "top", "middle", "bottom", or "side" of a particular column is relative because the actual position at which material is to be introduced or recovered is dependent on the conditions being maintained in the particular column. For example, a stream entering the "bottom" of a column may actually enter several stages above the sump including the reboiler of the column, and a line/stream exiting the "top" of the column may actually exit several stages below the top stage including the condenser of the column. Thus, such terms herein are included for ease of reference to describe a general orientation regarding various columns and lines/streams and such terms are not meant to be limiting to one exact location. Also, the columns referenced herein can be interconnected by a series of feed/recycle lines which serve to transport streams comprising reactants and/or products. The direction of flow for each line is indicated in, e.g., FIGS. 1 and 2. Various valves, heaters, and other fittings, optionally, can be included with the feed/recycle lines shown in FIGS. 1 and 2 in adapting the design to a particular installation. Also, although for illustrative purposes, the Figures and their description may depict singular vessels, such as reaction vessels or mixing vessels, it is understood that multiple vessels in series or parallel may be used where suitable.

FIG. 1 illustrates a dialkyl carbonate production/recovery plant design/apparatus 400 in accordance with embodiments described herein. The plant 400 includes a reaction section 420 and a separation section 430. With reference to the reaction section 420, stream 409 comprises oxygen (O₂), which can be provided in any form, such as gaseous form. Oxygen sources include, for example, air, or oxygen-containing gases including those having greater than or equal to 95 weight percent molecular oxygen, specifically greater than or equal to 99 weight percent molecular oxygen. Oxygen containing gases are commercially available from, for example, Air Products.

Stream 407 comprises carbon monoxide (CO) which can be provided in any form, such as gaseous form being especially suitable. Carbon monoxide sources include, for example, carbon monoxide, carbon dioxide mixtures, syngas and/or other carbon monoxide-containing gases and combinations thereof, including those having greater than or equal to 95 weight percent (wt%) molecular carbon monoxide, specifically greater than or equal to 99 weight percent molecular carbon monoxide.

Stream 413 (first alkanol stream) comprises alkanol, such as methanol (MeOH). It is noted, however, that other alkanols could be used. Examples of alkanols include primary, secondary, and tertiary C₁-C₁₂ alkanols, specifically, primary C₁-C₆ alkanols.

The first alkanol stream 413, oxygen stream 409 and carbon monoxide stream 407 can be added to a reactor (e.g., stirred tank reactor 50), e.g., in a molar ratio of 0.5 to 0.7 (alkanol): 0.04 to 0.06 (oxygen): 0.8 to 1.2 (carbon monoxide) respectively, and specifically, greater than or equal to 0.6 (alkanol): greater than or equal to 0.05 (oxygen): greater than or equal to 1 (carbon monoxide). Catalyst stream 405 comprising a catalyst, can also be added to stirred tank reactor 50 upon demand, and/or the catalyst could remain in the stirred tank reactor 50. Examples of catalysts include those capable of catalyzing the reactants entering reactor 50 to produce a dialkyl carbonate formation reaction, as is known in the art. The catalyst can comprise iron, copper, nickel, cobalt, zinc, ruthenium, rhodium, palladium, silver, cadmium, rhenium, osmium, iridium, platinum, gold, mercury, and so forth, and combinations comprising at least one of the foregoing metals. As an example, the catalyst can comprise copper and chloride ions in a molar ratio of Cu : Cl of between 0.1 to 3, e.g., between 0.5 to 1.5, specifically a molar ratio of Cu : Cl of greater than or equal to 0.8, and more specifically a molar ratio of Cu : Cl of small than or equal to 1.5. Further examples of catalysts include cuprous chloride (CuCl) and cupric chloride (CuCl₂). It is noted that the amount of catalyst used relative to the reactants (e.g., alkanol, carbon monoxide, oxygen and hydrochloric acid) can depend on the particular catalyst employed. The catalyst concentration should be sufficiently high to produce an acceptable yield, but should be kept below a concentration that would cause solid setting of the catalyst in the reactor 50 or clogging of the equipment. For example, when the catalyst comprises CuCl, the catalyst concentration can be 50 to 250 grams/liter of total liquid reaction volume of the mixture in reactor 50, especially 80 to 180 grams/liter of total liquid reaction volume of the mixture in reactor 50.

During operation of the DMC production process, an effective chloride ion concentration can be maintained by the addition hydrochloric acid (HCl) from stream 406. Sufficient HCl from stream 406 can be added from a HCl tank (not shown) during the course of the reaction to maintain a molar ratio of Cu : Cl of greater than or equal to one, for example. The concentration of HCl can be continuously determined and controlled by the addition of, e.g., fresh HCl (stream 406). A typical mass ratio for HCl feed to total liquid feed (e.g., the sum of streams 431+413+406+164) entering reactor 50 is 4 x 10⁻⁴ to 6 x 10⁻³.

Streams 406, 413, 407, 409, 431, 417, 164, and 405 can enter the reactor 50, as shown in FIG. 1, where the constituents react to produce a stream 403 (second alkanol stream) exiting the reactor 50. With reference to FIG. 1, the catalyzed reaction of alkanol, oxygen, and carbon monoxide may be performed in a single reactor 50, or in two or more reactors 50. The conditions in reactor 50 can be selected to balance the maximizing of the yield of dialkyl carbonate while minimizing the degradation of dialkyl carbonate. The reaction can be performed in a single reactor 50, at a temperature of 50°C to 250°C. Within this range, the temperature can be at least 100°C. Also within this range, the temperature may be up to 150°C. The reactor 50 can be maintained at a pressure of 15 to 35 bar gauge (barg). Within this range, a pressure of at least 20 barg can be employed. Also within this range, a pressure up to 28 barg can be employed. In the case of dual reactor systems, the catalyst may be recycled between tanks. Stream 403 can comprise dialkyl carbonate, alkyl chloroformate, hydrochloric acid, water, carbon monoxide, alkanol, oxygen, inert gases coming with fresh oxygen and carbon monoxide streams as well as side products, such as alkyl chlorides, carbon dioxide and dialkyl ethers. Examples of dialkyl carbonates are those that include a carbonate group disposed between two alkyl groups. The dialkyl carbonate formation reaction will create a dialkyl carbonate that is dependent upon the alkanol used as a reactant. Thus, if methanol is used as a reactant in stream 413, for example, the dialkyl carbonate will comprise dimethyl carbonate, and so forth.

Stream 403 is typically withdrawn from the reactor 50 in a gas/vapor form. The term "vapor" herein refers to gaseous organic components of the mixture, such as for example, evaporated dialkyl carbonates, alcohols, alkyl chloroformates, and so forth, and to water vapor. That is, the term "vapor" refers to fluids having a boiling point of greater than or equal to -50°C at atmospheric pressure. In contrast, the term "gas" herein refers to, e.g., gaseous oxygen, carbon dioxide, carbon monoxide and optional nitrogen. That is, the term "gas" refers to such constituents having a boiling point of less than -50°C at one atmosphere pressure. The vapor in stream 403 can be at least partially condensed in a condenser 52 (e.g., at least 10% by weight) or more fully condensed (e.g., at least 90% by weight) and fed to a first gas-liquid separator 90. The reaction section 420 of plant 400 can optionally employ a single gas-liquid separator or a plurality of (i.e., at least 2; and in a more specific embodiment up to, e.g., 5) gas-liquid separator. The first gas-liquid separator 90 can be maintained at a pressure within 10 percent (%), specifically within 1%, of the pressure of the reactor 50. The gas effluent from the first gas-liquid separator 90 (e.g., flash separator), stream 408, comprising unreacted carbon monoxide and oxygen, by produced carbon dioxide and low boiling point byproducts (low boiling organics) can partially enter a separation section 54 and the remaining stream can merge with the output as stream 417, which merges with stream 407 to return excess carbon monoxide to reactor 50. Stream 410 is a purge stream comprising low boiling organics. Specifically, the low boiling organics, as referred to herein, can comprise organic compounds having a boiling point less than or equal to 65°C, such as, for example, methylal, dimethyl ether, methyl chloride, and a combination of any of the foregoing. Stream 414, comprising carbon monoxide and carbon dioxide free from low boiling point organic compounds, can be sent to a proper treatment unit to recover and reutilize the carbon monoxide.

Stream 404 comprising dialkyl carbonate, hydrochloric acid, water, low boiling organics, unreacted alkanol, alkyl chloroformate and carbon monoxide can then exit the separator 90, as shown in FIG. 1, and enter a second gas-liquid separator 100 (e.g., flash separator), which can be maintained at a pressure less than or equal to 20% of the pressure of reactor 50 (e.g., less than or equal to 3 bar gauge (300 kPa), specifically less than or equal to 0.2 bar gauge (20 kPa)) to achieve a separation of at least 90 wt%, specifically at least 95 wt%, by weight of gas in stream 404. In an embodiment, substantially all of the gas is removed from the mixture. The gas effluent removed from the second gas-liquid separator 100 can also be recycled. The vapor in the mixture can be in an at least partially condensed form (i.e., at least 10% condensed), especially a fully condensed form (i.e., at least 90% condensed), before entering the first gas-liquid separator 90, and between the first gas-liquid separator 90 and the second gas-liquid separator 100. The vapor in stream 404 can be at least partially condensed (e.g., at least 10 wt%) or more fully condensed (e.g., at least 90 wt%) prior to entering the second gas-liquid separator 100). According to embodiments, all of the gas can be removed in the second gas-liquid separator 100. The gas effluent from the second gas-liquid separator 100, stream 411, comprising unreacted carbon monoxide, carbon dioxide and other organic low boiling point components (low boiling organics), also can be treated in separation unit 54 to recover unreacted carbon monoxide.

Stream 401 exiting the second gas-liquid separator 100 can comprise a single liquid phase comprising dialkyl carbonate, residual methanol, water, low boiling organics, hydrochloric acid, and alkyl chloroformate. The alkyl chloroformate is then removed from the stream 401. For example, stream 401 can enter fluid passageway 110 configured to remove alkyl chloroformate by decomposition into methanol, carbon dioxide, dialkyl carbonate and hydrochloric acid. U.S. Patent No. 6,784,277 describes benefits and methods of removing alkyl chloroformate using a fluid passageway.

After removal of the alkyl chloroformate from the stream 401 in unit 110, the stream can proceed (e.g., as stream 402, comprising dialkyl carbonate, water, low boiling organics and hydrochloric acid) through heat exchanger 150 to at least partially vaporize stream 402. Heat exchanger 150 can have a residence time of less than or equal to 10 minutes. Stream 402 can then enter acid removal column (crude product separation column) 160 to optionally remove HCl. Column 160 also can help remove any entrained catalyst (e.g., CuCl) in stream 403, which could otherwise contribute to downstream corrosion. In the column 160, the vaporized condensate can encounter a counter-flowing liquid supplied by counter-flowing liquid line (not shown), which can comprise an alkanol and dialkyl carbonate azeotrope mixture, to a higher point in the column 160 (e.g., upper third). The counter-flowing liquid can trap any remaining HCl, which may be removed, e.g., from the bottom, of column 160 in stream 164 comprising hydrochloric acid, water and small amounts of alkanol and dialkyl carbonate (e.g., less than or equal to 10 percent (%) by weight (wt.)). For example, stream 164 can comprise at least 6 weight percent HCl and less than or equal to 15 weight percent organic compounds. Hydrochloric acid can be recycled back to reactor 50 with use of HCl recycle stream 164 and merge with fresh HCl feed stream 406. Optionally, a portion of HCl recycle stream 164 can be passed through a heat exchanger and recycled to column 160.

Stream 412 comprising dialkyl carbonate, alkanol (e.g., methanol), water, and low boiling organics (low boiling byproducts comprising organics having a boiling point less than or equal to 65°C) can be removed, e.g. from the top, of column 160 and advantageously passed into a first dividing wall distillation column (DWC1) 200, as shown in FIG. 1.

According to some exemplary embodiments, the use of DWC1 200 can reduce the number of distillation columns needed to produce a final dialkyl carbonate product, such as DMC, while maintaining the purity level (e.g., greater than or equal to 99.8 wt% purity), thereby significantly reducing the plant investment and overall cost of production, and without increasing energy consumption. For example, FIG. 2 schematically depicts a DMC/methanol/water separation system 300 comprising three distillation columns: a first distillation column 301, a second distillation column 302, and a third distillation column 303. FIG. 2 also depicts a separator 601. This separator 601 is intended to be a unit to separate an aqueous-rich phase and an organic-rich phase and it can be for example a decanter, pervaporation unit, and so forth. Decanter 601 is referred to herein as a non-limiting example of this separator. According to some embodiments, the use of DWC1 200 can eliminate the need for at least the DMC recovery distillation column 302 of FIG. 2, as described in further detail below, without increasing the energy consumption of the entire system and reducing equipment and production costs.

With reference to FIG. 2, a mixed feed stream 412 comprising dialkyl carbonate, alkanol (alkyl alcohol), water and low boiling organics (low boiling byproducts comprising organic compounds having a boiling point less than or equal to 65°C) can be removed from the top of column 160 (shown in FIG. 1). As shown in FIG. 2, stream 412 can thus comprise methanol (MeOH), DMC, water and low boiling organics and be separated into three product streams comprising: mixture of alkanol/dialkyl carbonate azeotrope (methanol/DMC azeotropic composition) and low boiling organics (stream 312), dialkyl carbonate (DMC, stream 314) and water (stream 316). Feed stream 412 can enter the first distillation column 301, which can distill out a product stream 312 comprising an azeotropic composition of alkanol and dialkyl carbonate (i.e., azeotrope methanol/dimethyl carbonate, and the low boiling organics (organics having a boing point less than or equal to 65°C)). The stream 315 exiting column 301, which can comprise a mixture of dialkyl carbonate and water, with small amounts of alkanol (methanol), can enter decanter 601. Stream 315 can be decanted into a dialkyl carbonate rich stream 317 (organic phase) and a water rich (light aqueous phase) stream 318. Stream 317 comprising dialkyl carbonate can enter dialkyl carbonate recovery column 302, where stream 320 comprising dialkyl carbonate (e.g., DMC), some alkanol (methanol), and low boiling organics and water can be condensed and recycled to the decanter 601. Also exiting column 302 is product stream 314 comprising DMC.

Stream 318 exiting the decanter 601 can be fed to waste water recovery column 303. From column 303, azeotrope DMC/water can be recycled, e.g., from near the top (distillate of the column) in stream 324 to the decanter 601 and waste water stream 316 can be removed, e.g., the bottom residue stream of column 303. Bleedstream 326 comprising water, dialkyl carbonate, alkanol and low boiling organics and coming off stream 318 can be fed back to column 301, as shown in FIG. 2. Use of stream 326 can help avoid buildup of low boiling organics and alkanol (e.g., methanol) in decanter 601.

Thus, while the above-described process and design of FIG. 2 could be employed to produce DMC, three distillation columns 301, 302, and 303 are needed. Moreover, it is noted that gradually purer methanol could be obtained from column 301 overheads (stream 312) by progressively increasing the operating pressure of column 301. In this method, a stream 312 comprising 93 wt% methanol, for example, could be obtained from column 301 in stream 312, with column 301 operating at a pressure greater than or equal to 8 bar gauge (800 kPa), for example. Thus, stream 312 comprising a greater weight percent methanol (purer methanol) could be obtained by operating at a higher pressure, but at the cost of also using higher pressure steam as a heating medium in the process. Moreover, an additional drawback if column 301 operates at higher pressure is that an increase in the number of theoretical stages in the column 301 would be needed to minimize the presence of methanol in the exiting stream 315 sent to decanter S610. It would thus be desirable to reduce at least one of the columns 301, 302, 303 and increase the overall energy efficiency of the production, as well as reduced the costs associated with the DMC production.

According to some embodiments, the need for at least column 302 can be eliminated while producing a purified dialkyl carbonate product, specifically purified DMC (impurities in trace amounts; i.e., less than 0.01 wt% based upon the total weight of the purified dialkyl carbonate product).

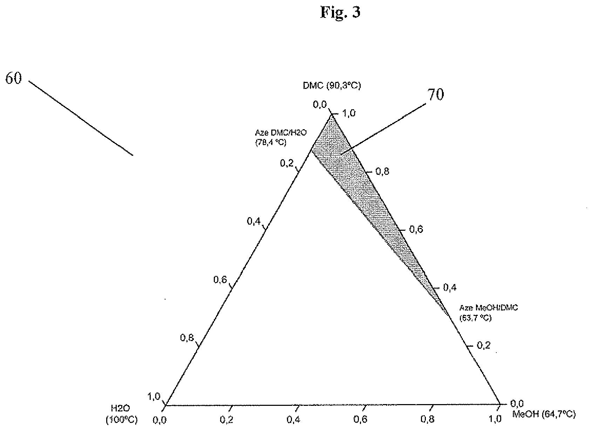

As shown in FIG. 1, effluent reactant stream 412 can enter DWC1 200. According to some exemplary embodiments, a mixture comprising DMC, methanol and water, which also can include some low boiling organics, can be separated into pure DMC, an azeotropic mixture of MeOH/DMC with low boiling organics and a water stream that can be purged. This can be accomplished for mixture feeds (e.g., stream 412) having a composition, for example, within the triangle 70 formed in a ternary composition diagram between three vertices: pure methanol, azeotrope methanol/DMC and azeotrope DMC/water, shown in FIG. 3. FIG. 3 depicts a mass ternary composition diagram 60 of a DMC/methanol/water ternary system. The shadowed triangle 70 of FIG. 3 shows examples of effective ternary feeds at atmospheric pressure. More specifically, the compositional ranges of the feed can comprise, for example, 100 wt% DMC (pure dimethyl carbonate); 68 wt% methanol and 32 wt% methanol/DMC azetrope; and 87 wt% DMC and 13 wt% azeotrope DMC/water). In other words, the compositional ranges lie within region 70, the area of the ternary diagram identified by coordinates: 100 wt% DMC; 68 wt% methanol and 32 wt% DMC; and 87 wt% DMC and 13 wt% water. The amount of the referenced low boiling organics is considered low enough not to influence the ternary equilibrium of the DMC/methanol/water system.

Thus, as shown in FIG. 1, effluent reactant stream 412 (distillate stream) can exit column 160 to enter DWC1 200 and can comprise dialkyl carbonate, water, alkanol and low boiling organics (low boiling point organics). Specifically, the low boiling organics can comprise organic compounds having a boiling point less than or equal to 65°C, such as, for example, methylal, dimethyl ether, methyl chloride, and a combination of any of the foregoing. The low boiling organics are typically present in stream 412 in a total amount less than or equal to 2 weight percent based upon the total weight percent of stream 412, specifically less than 1 weight percent.

Regarding the structure of DWC1 200, as an example, this dividing wall distillation column can comprise the following sections: a) an inflow section and b) an offtake section, and d) a lower column section of, e.g., greater than or equal to four, e.g., greater than or equal to eight, theoretical stages, e) a condenser and f) a reboiler. The inflow section and the offtake section can both comprise, greater than or equal to ten, specifically, greater than or equal to 20, more specifically, greater than or equal to twenty-six, theoretical stages, and can be separated by a wall (e.g., a vertical wall). The upper column section can have greater than or equal to four, specifically, greater than or equal to eight, theoretical stages. The lower column section can have greater than or equal to four, specifically, greater than or equal to eight, theoretical stages. This design was employed in Examples 1 and 2 referenced below. As explained by Schultz et al., Chem. Eng. Process., May 2002, pages 64-70, a basis for a dividing wall column concept is the Petlyuk's configuration but with the two distillation columns installed within a single shell.

A divided wall distillation column, such as DWC1 200, can comprise a dividing wall 210, as schematically shown in FIG. 1, which can vertically bisect a portion of the interior of the column 200. It is not necessary for the dividing wall 210 of the dividing wall distillation column 200 to extend all the way to the top and/or bottom sections of the column 200, as shown in FIG. 1, thus enabling the dividing wall distillation column 200 to be reboiled and refluxed. Such a divided wall distillation column 200 can enable an inlet stream to enter on, e.g., one side of the dividing wall 210 of the column 200, and one or more exiting streams, e.g. side drawn streams, can be located on the other side of the dividing wall 210. Such a design can increase the stability of the column 200 and enable one or more product streams of differing composition to exit the dividing wall column 200.

With further reference to FIG. 1, a stream 431 comprising alkanol, dialkyl carbonate, water and low boiling organics can be recovered from, e.g., the top (distillate) of DWC1 200. Specifically, the low boiling organics in this stream can comprise organic compounds having a boiling point less than or equal to 65°C, such as, for example, methylal, dimethyl ether, methyl chloride, and a combination of any of the foregoing. The low boiling organics can be present in stream 431 in a total amount less than or equal to 2 weight percent based upon the total weight percent of stream 431, specifically less than 1 weight percent. Stream 431 also can comprise DMC/methanol azeotrope. Stream 431 comprising alkanol and dialkyl carbonate, can be partly recycled back to the original alkanol feed stream 413. A stream 426 comprising dialkyl carbonate, such as pure DMC (impurities only in trace amounts) can be recovered from, e.g., the bottom of DWC1 200. The DWC1 200 can operate at, for example, atmospheric pressure or above atmospheric pressure. The temperature profile of the DWC 1 200 can be greater than or equal to 60°C, specifically greater than or equal to 70°C. Examples of temperature ranges for the temperature profile in DWC1 200 are 60°C to 1840°C, specifically 70°C to 1440°C. The operating pressure in DWC1 200 can be greater than or equal to 1 bar (100 kPa), specifically greater than or equal to 1.1 bar (110 kPa). Examples of pressure are 1.2 to 3 millibar (120 to 300 Pa).

Stream 416, which can comprise dialkyl carbonate, water and traces of alkanol, and exiting DWC1 200 from the side, for example, also can be recovered in the process. Stream 416 can enter decanter S610 and be split into two phases: stream 418 (organic phase) and stream 421 (aqueous phase). Stream 418 comprises mostly dialkyl carbonate, some water and traces alkanol, which is returned back to the DWC1 200, such as to the top stage of the lower section of the column 200. Stream 421 (aqueous phase), which can comprise mostly water, some dialkyl carbonate and traces of alkanol can exit the decanter S610, for example as a side drawn, and be sent to waste water recovery column 630. A purge stream from column 630 can be included such as, for example, a bottom residue stream 422, which can comprise mostly water (greater than or equal to 95 wt% based upon the total weight of stream 422, specifically, greater than or equal to 99 wt%) and alkanol (less than or equal to 5 wt% based upon the total weight of the stream 422, specifically, less than or equal to 1 wt%). The temperature profile of column 630 can be greater than or equal to 60°C, specifically greater than or equal to 70°C. Examples of temperature ranges for the temperature profile in the column 630 include 60°C to 120°C, specifically 70°C to 110°C. The operating pressure in column 630 can be greater than or equal to 0.5 bar (50 kPa), specifically greater than or equal to 0.8 bar (80 kPa). Examples of pressure include 0.8 to 3 mbar (80 to 300 kPa).

Stream 424, which can comprise dialkyl carbonate, water, and alkanol, can be recycled from the top of column 630 back to the decanter S610. Product stream 426, which can comprise pure dialkyl carbonate, such as pure DMC with impurities only in trace amounts can be recovered from, for example, the bottom of DWC1 200. As further shown in FIG. 1, stream 426 can optionally proceed through a storage tank 429 for collection of DMC product. From storage tank 429, DMC product can be partially returned to DWC1 200 during startup of the plant or when adjustment of feed composition into the desired range of operation is needed (stream 427), or be used elsewhere, e.g., for the production of diphenyl carbonate (DPC) (from stream 428).

In an embodiment, a method of producing dialkyl carbonate: introducing a reactant stream (412) comprising dialkyl carbonate, water and alkanol to a first divided wall distillation column (200); separating the mixture with use of the divided wall distillation column (200); and recovering a product stream (426) comprising dialkyl carbonate.

In an embodiment, a method of producing dialkyl carbonate can comprise: reacting oxygen, alkanol, carbon monoxide, hydrochloric acid and a catalyst in a reactor to produce an alkanol stream; separating constituents of the alkanol stream in a separator; and recovering a dialkyl carbonate stream. The method also comprises introducing the dialkyl carbonate stream to an acid removal distillation column; and recovering from the acid removal distillation column a reactant stream comprising a mixture of dialkyl carbonate, water and alkanol. The method further comprises introducing the reactant stream to a first divided wall distillation column; separating the mixture with use of the divided wall distillation column; and recovering a product stream comprising dialkyl carbonate.

In the various embodiments: (i) the dialkyl carbonate can comprise dimethyl carbonate, the alkanol comprises methanol, and the product stream (426) comprises purified dimethyl carbonate having a purity of greater than 99.9 wt% based upon a total weight of the product stream; and/or (ii) the method can further comprise adjusting concentration of reactants in the reactant stream (412) by directing a recycle stream (427) comprising purified dimethyl carbonate from the product stream (426) to the reactant stream (412) upstream of the reactant stream (412) entering the divided wall distillation column (200); and/or (iii) the method can further comprise recovering a first stream (431) comprising dialkyl carbonate, water, and alkanol from the divided wall distillation column (200); and/or (iv) the method can further comprise recovering a second stream (416) comprising dialkyl carbonate, water, and alkanol from divided wall distillation column (200), introducing the second stream (416) into a separator (S610), recovering from the separator (S610) a third stream (418) comprising dialkyl carbonate, and recycling the third stream (418) to the divided wall distillation column (200); and/or (v) the method can further comprise recovering from the separator (S610) a fourth stream (421) comprising water, alkanol, and dialkyl carbonate, introducing the fourth stream (421) into a water recovery distillation column (630), recovering from the water recovery distillation column (630) a fifth stream (424) comprising water, alkanol, and dialkyl carbonate, and a sixth stream (422) comprising water, and recycling the fifth stream (424) to the separator (S610); and/or (vi) the first stream (431) can comprise dimethyl carbonate/methanol azeotrope; and/or (vii) the method can further comprise recovering a first stream (431) comprising dialkyl carbonate and alkanol from the divided wall distillation column (200), and recycling the first stream (431) to the reactor; and/or (viii) the method can further comprise reacting oxygen, alkanol, carbon monoxide, hydrochloric acid and a catalyst in a reactor to produce an alkanol stream (403), separating constituents of the alkanol stream (403) in a separator (90 and/or 100), and recovering a dialkyl carbonate stream (402) from the separator (90 and/or 100) and introducing the dialkyl carbonate stream (402) to an acid removal distillation column (160); and/or (ix) the separator (S610) can be a decanter.

Also included herein are methods for producing diphenyl carbonate, comprising reacting the purified dimethyl carbonate of any of the above methods to produce the diphenyl carbonate. Similarly included are methods for producing bisphenol-A polycarbonate, comprising: melt polymerizing the diphenyl carbonate to produce the bisphenol-A polycarbonate.

In an embodiment, an apparatus configured for separating a mixture comprising dialkyl carbonate, water and alkanol, can comprise: a first divided wall distillation column (200) connected to an input line for introduction of reactants, the reactants comprising dialkyl carbonate, water and alkanol; the first divided wall distillation column (200) comprising a product line to provide a product stream (426) comprising dialkyl carbonate; a separator (S610) connected to the divided wall distillation column (200); and a water recovery distillation column (630) connected to the separator (S610); wherein the apparatus comprises a plurality of lines configured to transport streams between at least two of the first divided wall distillation column (200), the separator (S610) and the water recovery distillation column (630).

In the various embodiments: (i) the first dividing wall distillation column (200) can be connected to the input line for introduction of the reactants, and to a first line, a second line and the product line, the first line extends from the top of the first dividing wall distillation column (200) to provide a first stream (431) comprising dialkyl carbonate, water, and alkanol, the second line extends from the side of the first dividing wall distillation column (200) to the separator S(610) to provide a second stream (416) comprising dialkyl carbonate, water and alkanol, and the product line extends from the bottom of the first dividing wall distillation column (200) to provide a product stream (426) comprising dialkyl carbonate; the separator (S610) is connected to a third line extends from the bottom of the separator (S610) to the first dividing wall distillation column (200) to provide a third stream (418) comprising dialkyl carbonate, and a fourth line extends from the side of the separator (S610) to a water recovery distillation column (630) to provide a fourth stream (421) comprising water; and the water distillation column (630) is connected to a fifth line to provide a fifth stream (424) comprising water, alkanol and dialkyl carbonate to be recycled back to the separator (S610); and/or (ii) the dialkyl carbonate can comprise dimethyl carbonate, the alkanol comprises methanol, and the product stream (426) comprises purified dimethyl carbonate having a purity of greater than 99.9 wt% based upon a total weight of the product stream.

Methods and apparatuses described above are further illustrated by the following non-limiting examples. It is noted that Examples 1 and 2 below are based, in part, on computer simulation for a proposed production facility (e.g., FIG. 1).

### EXAMPLE 1

In this example and with reference to FIG. 1, effluent reactant stream 412 comprising 79,995 kilograms/hour (kg/h) (mixture of 69.8 wt% DMC, 27.6 wt.% MeOH, 2.4 wt% water, 0.2 wt% methylal, 0.06 wt% dimethyl ether and 0.04 wt% methyl chloride) was introduced to DWC1 200 operating at 1 bar absolute (bara). DWC1 200 comprised the following sections: a) an inflow section and b) an offtake section, both with twenty-six trays and separated by a vertical wall, c) an upper column section of eight trays, and d) a lower column section of eight trays, e) a condenser, and f) a reboiler.

Overhead flowrate (stream 431) from the DWC1 column 200 was 36,909 kg/h with a composition of 59.8 wt% MeOH, 38.9 wt% DMC, 0.7 wt% water, 0.4 wt% methylal, 0.1 wt% dimethyl ether and 0.08 wt% methyl chloride. Temperature profile of this column ranges from 90°C in the bottom to 62°C in the distillate. Reflux ratio of this column is 1.5. A side-draw (stream 416) of 17,853 kg/h was taken from the offtake section of the DWC1 200 at the 4^{th} stage of this section (counting from bottom to top) with a composition of 88.4 wt% DMC, 11.6% wt% water and 0.05 wt% MeOH. This stream 416 was decanted in decanter 610 and split in two phases at 30°C. In particular, 16,226 kg/h of organic phase (97.3 wt% DMC, 2.7 wt% water and 0.05 wt% MeOH) was returned back to the top stage of the lower section of the DWC1 200 by stream 418. Then, 1,931 kg/h of aqueous phase (86.5 wt% water, 13.2 wt% DMC and 0.3 wt% MeOH) was sent to the wastewater recovery column (630) by stream 421. Column 630 purged 1,627 kg/h of 99.97 wt% water and 0.03 wt% MeOH as a bottom residue in stream 422, while 304 kg/h of 83.6 wt% DMC, 14.6 wt% water and 1.8 wt% MeOH was recycled back to the decanter 610 by stream 424. Column 630 operates at 1 bar and a temperature range from 100°C to 77°C. Reflux ratio is 0.4. From stream 426, 41,460 kg/h of pure DMC product was obtained from the bottom of the DWC1 200 with impurities just in trace amounts.

### EXAMPLE 2

In this example and with further reference to FIG. 1, stream 412 comprising 35,315 kg/h (48.9 wt% MeOH, 45.1 wt% DMC, 5.4 wt% water, 0.4 wt% methylal, 0.1 wt% dimethyl ether and 0.09 wt% methyl chloride) was obtained from the top of the column 160. This composition lies outside of the boundaries of triangle 70 in Figure 3. In order to adjust the composition of the 412 stream into the acceptable range of compositions to properly operate the divided wall column DWC1 200, 13,512 kg/h of recycled, pure DMC (impurities in trace amounts only) from recycle stream 427 were blended together with stream 412 and fed to DWC1 200. This mixed stream of 48,828 kg/h (60.3 wt% DMC, 35.3 wt% MeOH, 3.9 wt% water, 0.3 wt% methylal, 0.09% wt% dimethyl ether and 0.06 wt% methyl chloride) was introduced to DWC1 200 operating at the same pressure as in Example 1. The DWC1 200 comprised the following sections: a) an inflow section and b) an offtake section, both with twenty-six trays and separated by a vertical wall, c) an upper column section of eight trays, d) a lower column section of eight trays, e) a condenser, and f) a reboiler.

Overhead flowrate (stream 431) from the DWC1 200 was 26,983 kg/h with a composition of 64.0 wt% MeOH, 35.0 wt% DMC, 0.2 wt% water, 0.6 wt% methylal, 0.2 wt% dimethyl ether and 0.1 wt% methyl chloride. A side-draw (stream 416) of 21,950 kg/h was taken from the offtake section of the DWC1 200 at the 4^{th} stage of this section (counting from bottom to top) with a composition of 89.1 wt% DMC, 10.9 wt% water and 0.08 wt% MeOH. Temperature profile of this column ranges from 90°C in the bottoms to 62°C in the distillate. Reflux ratio of this column is 5. Stream 416 was decanted in decanter S610 and split in two phases, an organic phase (stream 418) and an aqueous phase (stream 421). In particular, 20,111 kg/h of organic phase in stream 418 (97.2 wt% DMC, 2.7 wt% water and 0.08 wt% MeOH) was returned back to the top stage of the lower section of the DWC1 200. Regarding the aqueous phase, 2,187 kg/h (86.2 wt% water, 13.3 wt% DMC and 0.5 wt% MeOH) was sent to distillation column 630 (wastewater recovery column) by stream 421. Column 630 purged 1,839 kg/h of 99.9 wt% water and 0.06 wt% MeOH as a bottom residue (stream 422), while 348 kg/h of 83.4 wt% DMC, 13.7 wt% water and 2.8 wt% MeOH was recycle back to the decanter (S610) in stream 424. Column 630 operates at 1 bar and a temperature range from 100°C to 77 °C. Reflux ratio is 0.4. From stream 426, 20,006 kg/h of pure DMC product was obtained from the bottom of the DWC1 200 with impurities just in trace amounts.

As can be seen from the foregoing examples, pure DMC product (impurities in trace amounts) can be produced using a divided wall distillation column, which can reduce the number of columns needed, and thus reduce energy consumption and overall cost of production. For example, the divided wall column 200 (see FIG. 1), eliminates the need for distillation columns 301 and 302 (see FIG. 2). In other words, the divided wall column can replace two columns of another system, wherein the systems are otherwise the same.

Despite these processes, however, there is a continued need for methods and apparatuses exhibiting reduced energy consumption and requiring less processing equipment, specifically less distillation columns, while also capable of producing purified dialkyl carbonate, especially purified dimethyl carbonate.

In general, embodiments may alternately comprise, consist of, or consist essentially of, any appropriate components herein disclosed. The embodiments may additionally, or alternatively, be formulated so as to be devoid, or substantially free, of any components, materials, ingredients, adjuvants or species used in the prior art compositions or that are otherwise not necessary to the achievement of the function and/or objectives of the embodiments.

As used herein, a trace amount is an amount of less than 0.01 wt% based upon a total weight of the product. All ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combination" is inclusive of blends, mixtures, alloys, reaction products, and the like. Furthermore, the terms "first," "second," and the like, herein do not denote any order, quantity, or importance, but rather are used to distinguish one element from another. The terms "a" and "an" and "the" herein do not denote a limitation of quantity, and are to be construed to cover both the singular and the plural, unless otherwise indicated herein or clearly contradicted by context. The suffix "(s)" as used herein is intended to include both the singular and the plural of the term that it modifies, thereby including one or more of that term (e.g., the film(s) includes one or more films). Reference throughout the specification to "one embodiment", "another embodiment", "an embodiment", and so forth, means that a particular element (e.g., feature, structure, and/or characteristic) described in connection with the embodiment is included in at least one embodiment described herein, and may or may not be present in other embodiments. In addition, it is to be understood that the described elements may be combined in any suitable manner in the various embodiments.

## Claims

1. A method of producing a purified dialkyl carbonate, comprising:
introducing a stream (412) comprising dialkyl carbonate, water and alkanol to a first divided wall distillation column (200); and
separating the mixture with use of the divided wall distillation column (200); and
recovering a product stream (426) comprising the purified dialkyl carbonate from a bottom end of the divided wall distillation column (200);
recovering a first stream (431) comprising dialkyl carbonate, water, and alkanol from a top end of the divided wall distillation column (200);
recovering a second stream (416) comprising dialkyl carbonate, water, and alkanol from a side of the divided wall distillation column (200);
introducing the second stream (416) into a separator (S610);
recovering from a bottom of the separator (S610) a third stream (418) comprising dialkyl carbonate; and
recycling the third stream (418) to the side of the divided wall distillation column (200).

2. The method of Claim 1, wherein the dialkyl carbonate comprises dimethyl carbonate, the alkanol comprises methanol, and the product stream (426) comprises purified dimethyl carbonate having a purity of greater than 99.9 wt% based upon a total weight of the product stream.

3. The method of any of Claims 1-2, comprising adjusting concentration of components in the stream (412) by directing a recycle stream (427) comprising purified dimethyl carbonate from the product stream (426) to the stream (412) upstream of the stream (412) entering the divided wall distillation column (200).

4. The method of Claim 1, comprising:
recovering from the separator (S610) a fourth stream (421) comprising water, alkanol, and dialkyl carbonate;
introducing the fourth stream (421) into a water recovery distillation column (630);
recovering from the water recovery distillation column (630) a fifth stream (424) comprising water, alkanol, and dialkyl carbonate, and a sixth stream (422) comprising water; and
recycling the fifth stream (424) to the separator (610).

5. The method of Claim 1, wherein the first stream (431) comprises a dimethyl carbonate/methanol azeotrope.

6. The method of any of Claims 1-3, comprising:
recovering a first stream (431) comprising dialkyl carbonate and alkanol from the divided wall distillation column (200); and recycling the first stream (431) to a reactor.

7. The method of any of Claims 1-6, further comprising:
reacting oxygen, alkanol, carbon monoxide, hydrochloric acid and a catalyst in a reactor to produce an alkanol stream (403);
separating constituents of the alkanol stream (403) in a separator (90 and/or 100); and
recovering a dialkyl carbonate stream (402) from the separator (90 and/or 100) and introducing the dialkyl carbonate stream (402) to an acid removal distillation column (160); and
recovering the stream (412) from a top of the acid removal distillation column (160).

8. The method of any of Claims 1-7, wherein the separator (S610) is a decanter.

9. A method for producing diphenyl carbonate, comprising
reacting the purified dialkyl carbonate of any of Claims 1-8 to produce the diphenyl carbonate.

10. A method for producing bisphenol-A polycarbonate, comprising:
melt polymerizing the diphenyl carbonate of Claim 9 to produce the bisphenol-A polycarbonate.

11. An apparatus configured for separating a mixture comprising dialkyl carbonate, water and alkanol, comprising:
the first dividing wall distillation column (200) is connected to the input line for introduction of the feed, and to a first line, a second line and the product line, the first line extends from the top of the first dividing wall distillation column (200) to provide a first stream (431) comprising dialkyl carbonate, water, and alkanol, the second line extends from the side of the first dividing wall distillation column (200) to the separator (S610) to provide a second stream (416) comprising dialkyl carbonate, water and alkanol, and the product line extends from the bottom of the first dividing wall distillation column (200) to provide a product stream (426) comprising a purified dialkyl carbonate;
a separator (S610) connected to the divided wall distillation column (200); and
a water recovery distillation column (630) connected to the separator (S610); wherein the apparatus comprises a plurality of lines configured to transport streams between at least two of the first divided wall distillation column (200), the separator (S610) and the water recovery distillation column (630);
wherein the separator (S610) is connected to a third line extends from the bottom of the separator (S610) to the first dividing wall distillation column (200) to provide a third stream (418) comprising dialkyl carbonate.

12. The apparatus of Claim 11, wherein:
the separator (610) is connected to a fourth line extends from the side of the separator (S610) to a water recovery distillation column (630) to provide a fourth stream (421) comprising water; and
the water distillation column (630) is connected to a fifth line to provide a fifth stream (424) comprising water, alkanol and dialkyl carbonate to be recycled back to the separator (S610).

13. The apparatus of any of Claims 11-12, wherein the dialkyl carbonate comprises dimethyl carbonate, the alkanol comprises methanol, and the product stream (426) comprises purified dimethyl carbonate having a purity of greater than 99.9 wt% based upon a total weight of the product stream.

## Patentansprüche

1. Verfahren zum Herstellen eines gereinigten Dialkylcarbonats, umfassend:
Einleiten eines Stroms (412), der Dialkylcarbonat, Wasser und Alkanol umfasst, in eine erste Trennwand-Destillationskolonne (200); und
Trennen der Mischung unter Verwendung der Trennwand-Destillationskolonne (200); und Rückgewinnen eines Produktstroms (426), der das gereinigte Dialkylcarbonat von einem unteren Ende der Trennwand-Destillationskolonne (200) umfasst;
Rückgewinnen eines ersten Stroms (431), der Dialkylcarbonat, Wasser und Alkanol umfasst, von einem oberen Ende der Trennwand-Destillationskolonne (200);
Rückgewinnen eines zweiten Stroms (416), der Dialkylcarbonat, Wasser und Alkanol umfasst, von einer Seite der Trennwand-Destillationskolonne (200); Einleiten des zweiten Stroms (416) in einen Separator (S610); Rückgewinnen von einer Unterseite des Separators (S610) eines dritten Stroms (418), der Dialkylcarbonat umfasst; und
Rückführen des dritten Stroms (418) zu der Seite der Trennwand-Destillationskolonne
(200).

2. Verfahren nach Anspruch 1, wobei das Dialkylcarbonat Dimethylcarbonat umfasst, das Alkanol Methanol umfasst und der Produktstrom (426) gereinigtes Dimethylcarbonat mit einer Reinheit von mehr als 99,9 Gew.-%, bezogen auf das Gesamtgewicht des Produktstroms umfasst.

3. Verfahren nach einem der vorhergehenden Ansprüche 1 bis 2, umfassend das Einstellen der Konzentration von Komponenten in dem Strom (412) durch Leiten eines Rückführstroms (427), der gereinigtes Dimethylcarbonat umfasst, aus dem Produktstrom (426) zu dem Strom (412) stromaufwärts des Stroms (412), der in die Trennwand-Destillationskolonne ( 200) eintritt.

4. Verfahren nach Anspruch 1, umfassend:
Rückgewinnen von dem Separator (S610) eines vierten Stroms (421), der Wasser, Alkanol und Dialkylcarbonat umfasst;
Einleiten des vierten Stroms (421) in eine Wasserrückgewinnungs-Destillationskolonne (630);
Rückgewinnen von der Wasserrückgewinnungs-Destillationskolonne (630) eines fünften Stroms (424), der Wasser, Alkanol und Dialkylcarbonat umfasst, und eines sechsten Stroms (422), der Wasser umfasst; und
Rückführen des fünften Stroms (424) zum Separator (610).

5. Verfahren nach Anspruch 1, wobei der erste Strom (431) Dimethylcarbonat/Methanolazeotrop umfasst.

6. Verfahren nach einem der Ansprüche 1 bis 3, umfassend:
Rückgewinnen eines ersten Stroms (431), der Dialkylcarbonat und Alkanol umfasst, von der Trennwand-Destillationskolonne (200); und Rückführen des ersten Stroms (431) zu einem Reaktor.

7. Verfahren nach einem der Ansprüche 1 bis 6, ferner umfassend:
Umsetzen von Sauerstoff, Alkanol, Kohlenstoffmonoxid, Chlorwasserstoffsäure und eines Katalysators in einem Reaktor, um einen Alkanolstrom (403) zu erzeugen;
Trennen von Bestandteilen des Alkanolstroms (403) in einem Separator (90 und/oder 100); und
Rückgewinnen eines Dialkylcarbonatstroms (402) von dem Separator (90 und/oder 100) und Einleiten des Dialkylcarbonatstroms (402) in eine Säureentfernungs-Destillationskolonne (160); und
Rückgewinnen des Stroms (412) von einem oberen Ende der Säureentfernungs-Destillationskolonne (160).

8. Verfahren nach einem der Ansprüche 1 bis 7, wobei der Separator (S610) ein Dekanter ist.

9. Verfahren zum Herstellen von Diphenylcarbonat, umfassend Umsetzen des gereinigten Dialkylcarbonats nach einem der Ansprüche 1 bis 8, um das Diphenylcarbonat herzustellen.

10. Verfahren zum Herstellen von Bisphenol-A-Polycarbonats, umfassend:
Schmelzpolymerisieren des Diphenylcarbonats nach Anspruch 9 zur Herstellung des Bisphenol-A-Polycarbonats.

11. Vorrichtung, die zum Trennen einer Mischung konfiguriert ist, die Dialkylcarbonat, Wasser und Alkanol umfasst, umfassend:
die erste Trennwand-Destillationskolonne (200) ist mit der Eingangsleitung zur Einleitung der Beschickung verbunden, und mit einer ersten Leitung, einer zweiten Leitung und der Produktleitung, wobei sich die erste Leitung von der Oberseite der ersten Trennwand-Destillationskolonne (200) erstreckt, um einen ersten Strom (431) bereitzustellen, der Dialkylcarbonat, Wasser und Alkanol umfasst, wobei sich die zweite Leitung von der Seite der ersten Trennwand-Destillationskolonne (200) zum Separator (S610) erstreckt, um einen zweiten Strom (416) bereitzustellen, der Dialkylcarbonat, Wasser und Alkanol umfasst, wobei sich die Produktleitung von der Unterseite der ersten Trennwand-Destillationskolonne (200) erstreckt, um einen Produktstrom (426) bereitzustellen, der ein gereinigtes Dialkylcarbonat umfasst;
wobei ein Separator (S610) mit der Trennwand-Destillationskolonne (200) verbunden ist; und
eine Wasserrückgewinnungs-Destillationskolonne (630) mit dem Separator (S610) verbunden ist; wobei die Vorrichtung eine Mehrzahl von Leitungen umfasst, die konfiguriert sind, Ströme zwischen mindestens zwei der ersten Trennwand-Destillationskolonne (200), dem Separator (S610) und der Wasserrückgewinnungs-Destillationskolonne (630) zu transportieren;
wobei der Separator (S610) mit einer dritten Leitung verbunden ist, die sich von der Unterseite des Separators (S610) zu der ersten Trennwand-Destillationskolonne (200) erstreckt, um einen dritten Strom (418) bereitzustellen, der Dialkylcarbonat umfasst.

12. Vorrichtung nach Anspruch 11, wobei:
der Separator (610) mit einer vierten Leitung verbunden ist, die sich von der Seite des Separators (S610) zu einer Wasserrückgewinnungs-Destillationskolonne (630) erstreckt, um einen vierten Strom (421) bereitzustellen, der Wasser umfasst; und
die Wasserdestillationskolonne (630) ist mit einer fünften Leitung verbunden, um einen fünften Strom (424) bereitzustellen, der Wasser Alkanol und Dialkylcarbonat umfasst, um zurück zum Separator (S610) zurückgeführt zu werden.

13. Vorrichtung nach einem der Ansprüche 11 bis 12, wobei das Dialkylcarbonat Dimethylcarbonat umfasst, das Alkanol Methanol umfasst und der Produktstrom (426) gereinigtes Dimethylcarbonat mit einer Reinheit von mehr als 99,9 Gew.-%, bezogen auf das Gesamtgewicht des Produktstroms umfasst.

## Revendications

1. Procédé pour produire un carbonate de dialkyle purifié, comprenant :
l'introduction d'un flux (412) comprenant du carbonate de dialkyle, de l'eau et de l'alcanol à une première colonne de distillation à paroi divisée (200) ; et
la séparation du mélange en utilisant la colonne de distillation à paroi divisée (200) ; et la récupération d'un flux de produit (426) comprenant le carbonate de dialkyle purifié depuis une extrémité inférieure de la colonne de distillation à paroi divisée (200) ;
la récupération d'un premier flux (431) comprenant du carbonate de dialkyle, de l'eau et de l'alcanol depuis une extrémité supérieure de la colonne de distillation à paroi divisée (200) ;
la récupération d'un deuxième flux (416) comprenant du carbonate de dialkyle, de l'eau, et de l'alcanol depuis un côté de la colonne de distillation à paroi divisée (200) ;
l'introduction d'un deuxième flux (416) dans un séparateur (S610); la récupération depuis une partie inférieure du séparateur (S610) d'un troisième flux (418) comprenant du carbonate de dialkyle ; et
le recyclage du troisième flux (418) vers le côté de la colonne de distillation à paroi divisée (200).

2. Procédé selon la revendication 1, dans lequel le carbonate de dialkyle comprend du carbonate de diméthyle, l'alcanol comprend du méthanol, et le flux de produit (426) comprend du carbonate de diméthyle purifié ayant une pureté supérieure à 99,9 % en poids sur la base d'un poids total du flux de produit.

3. Procédé selon l'une quelconque des revendications 1-2, comprenant l'ajustement de la concentration de composants dans le flux (412) en orientant un flux de recyclage (427) comprenant du carbonate de diméthyle purifié depuis le flux de produit (426) vers le flux (412) en amont du flux (412) entrant dans la colonne de distillation à paroi divisée (200).

4. Procédé selon la revendication 1, comprenant :
la récupération depuis le séparateur (S610) d'un quatrième flux (421) comprenant de l'eau, de l'alcanol, et du carbonate de dialkyle ;
l'introduction d'un quatrième flux (421) dans une colonne de distillation de récupération d'eau (630) ;
la récupération depuis la colonne de distillation de récupération d'eau (630) d'un cinquième flux (424) comprenant de l'eau, de l'alcanol, et du carbonate de dialkyle, et un sixième flux (422) comprenant de l'eau ; et
le recyclage du cinquième flux (424) vers le séparateur (610).

5. Procédé selon la revendication 1, dans lequel le premier flux (431) comprend un carbonate de diméthyle/azéotrope de méthanol.

6. Procédé selon l'une quelconque des revendications 1-3, comprenant :
la récupération d'un premier flux (431) comprenant du carbonate de dialkyle et de l'alcanol depuis la colonne de distillation à paroi divisée (200) ; et le recyclage du premier flux (431) vers un réacteur.

7. Procédé selon l'une quelconque des revendications 1-6, comprenant en outre :
la réaction d'oxygène, d'alcanol, de monoxyde de carbone, d'acide hydrochlorique et d'un catalyseur dans un réacteur pour produire un flux d'alcanol (403) ;
la séparation de constituants du flux d'alcanol (403) dans un séparateur (90 et/ou 100) ; et
la récupération d'un flux de carbonate de dialkyle (402) depuis le séparateur (90 et/ou 100) et l'introduction du flux de carbonate de dialkyle (402) à une colonne de distillation d'élimination d'acide (160) ; et
la récupération du flux (412) depuis une partie supérieure de la colonne de distillation d'élimination d'acide (160).

8. Procédé selon l'une quelconque des revendications 1-7, dans lequel le séparateur (S610) est un décanteur.

9. Procédé pour produire du carbonate de diphényle, comprenant la réaction du carbonate de dialkyle purifié selon l'une quelconque des revendications 1-8 pour produire le carbonate de diphényle.

10. Procédé pour produire du polycarbonate de bisphénol-A, comprenant :
la polymérisation à l'état fondu du carbonate de diphényle selon la revendication 9 pour produire le polycarbonate de bisphénol-A.

11. Appareil configuré pour séparer un mélange comprenant du carbonate de dialkyle, de l'eau et de l'alcanol, comprenant :
la première colonne de distillation à paroi de division (200) est reliée à la ligne d'entrée pour l'introduction d'aliments, et à une première ligne, une deuxième ligne et la ligne de produit, la première ligne s'étend de la partie supérieure de la première colonne de distillation à paroi de division (200) pour fournir un premier flux (431) comprenant du carbonate de dialkyle, de l'eau, et de l'alcanol, la deuxième ligne s'étend du côté de la première colonne de distillation à paroi de division (200) au séparateur (S610) pour fournir un deuxième flux (416) comprenant du carbonate de dialkyle, de l'eau et de l'alcanol, et la ligne de produit s'étend de la partie inférieure de la première colonne de distillation à paroi de division (200) pour fournir un flux de produit (426) comprenant du carbonate de dialkyle purifié ;
un séparateur (S610) relié à la colonne de distillation à paroi divisée (200) ; et
une colonne de distillation de récupération d'eau (630) reliée au séparateur (S610) ; dans lequel l'appareil comprend une pluralité de lignes configurées pour transporter des flux entre au moins deux de la première colonne de distillation à paroi divisée (200), le séparateur (S610) et la colonne de distillation de récupération d'eau (630) ;
dans lequel le séparateur (S610) est relié à une troisième ligne qui s'étend de la partie inférieure du séparateur (S610) à la première colonne de distillation à paroi de division (200) pour fournir un troisième flux (418) comprenant du carbonate de dialkyle.

12. Appareil selon la revendication 11, dans lequel :
le séparateur (610) est relié à une quatrième ligne qui s'étend du côté du séparateur (S610) à une colonne de distillation de récupération d'eau (630) pour fournir un quatrième flux (421) comprenant de l'eau ; et
la colonne de distillation d'eau (630) est reliée à une cinquième ligne pour fournir un cinquième flux (424) comprenant de l'eau, de l'alcanol et du carbonate de dialkyle à recycler en retour vers le séparateur (S610).

13. Appareil selon l'une quelconque des revendications 11-12, dans lequel le carbonate de dialkyle comprend du carbonate de diméthyle, l'alcanol comprend du méthanol, et le flux de produit (426) comprend du carbonate de diméthyle purifié ayant une pureté supérieure à 99,9 % en poids sur la base d'un poids total du flux de produit.
